# EUROPEAN PATENT SPECIFICATION

(11) **EP 3 100 716 B1**
(45) Date of publication and mention of the grant of the patent: **26.09.2018**
(21) Application number: 16172302.8
(22) Date of filing: 28.03.2008
(51) Int. Cl.: A61K 8/44, A61K 8/20, A61Q 11/00, A61K 31/198, A61K 33/16, A61P 1/02, A61K 8/46, A61K 8/55, A61K 8/81

(54) **NOVEL SALTS AND THEIR USES**
NEUARTIGE SALZE UND DEREN VERWENDUNGEN
NOUVEAUX SELS ET LEURS UTILISATIONS

(30) Priority: 08.02.2008 US 27432 P; 08.02.2008 US 27420 P; 08.02.2008 US 27431 P; 09.02.2008 US 27442 P
(43) Date of publication of application: 07.12.2016
(62) Divisional of application: 08744602.7
(73) Proprietor: Colgate-Palmolive Company, New York, NY 10022 (US)
(72) Inventor: PRENCIPE, Michael, West Windsor NJ New Jersey 08550 (US); XU, Guofeng, Plainsboro, NJ New Jersey 08536 (US); SUBRAMANYAM, Ravi, Belle Mead, NJ New Jersey 08502 (US); WU, Donghui, Bridgewater, NJ New Jersey 08807 (US); CUMMINS, Diane, Livingston, NJ New Jersey 07039 (US); SULLIVAN, Richard, J., Atlantic Highlands, NJ New Jersey 07716 (US); SANTARPIA lll, Ralph Peter, Edison, NJ New Jersey 08820 (US); MELLO, Sarita Vera, North Brunswick NJ New Jersey 08902 (US); ZAIDEL, Lynette, Cranford, NJ New Jersey 07016 (US); CHOPRA, Suman, K., Monroe, NJ New Jersey 08831 (US); WANG, Qin, Monmouth Junction, NJ New Jersey 08852 (US); TAMBS, Gary, Edward, Belle Mead, NJ New Jersey 08502 (US); BARNES, Virginia, Monsul, Ringoes, NJ New Jersey 08551 (US); MORGAN, Andre, M., Robbinsville, NJ New Jersey 08691 (US); KOHLI, Rajnish, Hillsborough, NJ New Jersey 08844 (US); ROBINSON, Richard, Scott, Belle Mead, NJ New Jersey 08502 (US); LEITE, Sergio, Kendall Park, NJ New Jersey 08824 (US); SIMON, Eric, A., Somerset, NJ New Jersey 08873 (US)
(74) Representative: Wibbelmann, Jobst

(56) References cited:
- WO-A1-00/78270
- WO-A1-97/32565
- CN-A- 1 569 866
- FR-A1- 2 277 859
- GB-A- 2 354 441
- GB-A- 2 355 658
- JP-A- 2000 106 845
- JP-A- 2007 112 766
- US-A1- 2006 140 879
- US-A1- 2007 014 740
- KATAOKA S ET AL: "Buccal compsn. inhibiting tartar formation preventing gingivitis - contains arginate (salt) and water-soluble poly:phosphate(s) of specified average polymerisation deg", WPI/THOMSON,, vol. 1993, no. 12, 19 February 1993 (1993-02-19), XP002530591,

## Description

### CROSS-REFERENCE TO RELATED APPLICATIONS

This application claims the benefit of U.S. Ser. No. 61/027,442 filed February 9, 2008, U.S. Ser. No. 61/027,432 filed February 8, 2008, U.S. Ser. No. 61/027,431 filed February 8, 2008 and U.S. Ser. No. 61/027,420 filed February 8, 2008.

### FIELD OF THE INVENTION

This invention relates to novel salts of arginine and compositions comprising them.

### BACKGROUND OF THE INVENTION

Arginine and other basic amino acids have been proposed for use in oral care and are believed to have significant benefits in combating cavity formation and tooth sensitivity. Combining these basic amino acids with minerals having oral care benefits, e.g., fluoride and calcium, to form an oral care product having acceptable long term stability, however, has proven challenging. In particular, the basic amino acid may raise the pH and facilitate dissociation of calcium ions that can react with fluoride ions to form an insoluble precipitate. Moreover, the higher pH has the potential to cause irritation. At neutral pH or acidic pH, however, a system utilizing arginine bicarbonate (which the art teaches is preferred) may release carbon dioxide, leading to bloating and bursting of the containers. Moreover, it might be expected that lowering the pH to neutral or acidic conditions would reduce the efficacy of the formulation because the arginine may form an insoluble arginine-calcium complex that has a poorer affinity for the tooth surface, and moreover that lowering the pH would reduce any effect the formulation might have on buffering cariogenic lactic acid in the mouth. Partly because of these unaddressed formulation hurdles and partly because arginine has generally been viewed in the art as a potential alternative to fluoride rather than a co-active, there has been little motivation to make oral care products comprising both arginine and fluoride. Additional hurdles are potentially posed by addition of an antimicrobial agent. Commercially available arginine-based toothpaste, such as ProClude® and DenClude®, for example, contain arginine bicarbonate and calcium carbonate, but not fluoride nor any antimicrobial agent. Moreover, arginine salts have been used with cariostatic anions such as carbonate/ bicarbonate and phytate in oral care products for reducing dental caries (WO 97/32565).

At the same time, the value of antimicrobial agents, such as triclosan, in toothpaste has been recognized by many dentists. These agents however are challenging to deliver in effective amounts to the teeth and gums, and their solubility, delivery and retention on the teeth is formulation dependent. For example, triclosan (5-chloro-2-(2,4-dichlorophenoxy)phenol) is only slightly soluble in water.

Accordingly, there is a need for a stable oral care product providing arginine.

### SUMMARY OF THE INVENTION

It has been surprisingly discovered that these problems can be addressed using functional salts of arginine in oral care compositions as defined in the claims.

In other embodiments, the invention provides oral and personal care products comprising the salts of the invention, and methods of making and using the salts and products.

The salt can be used in compositions to promote oral health and/or systemic health, including cardiovascular health, e.g., by reducing potential for systemic infection via the oral tissues.

### DETAILED DESCRIPTION

The oral care compositions comprise a salt of arginine with a copolymer of methyl vinyl ether and maleic anhydride of a molecular weight of 30,000 to 800,000. This polymer comprises units formed of maleic anhydride and methyl vinyl ether, depicted at pH 7 as follows: 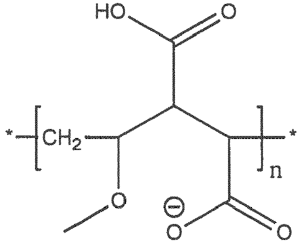 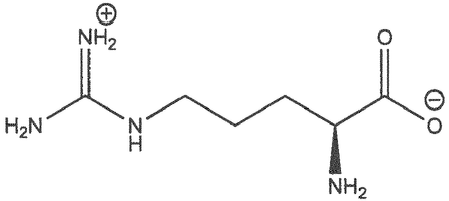 The methyl vinyl ether/maleic anhydride has a molecular weight (M.W.) of 30,000 to 800,000. In another embodiment, the methyl vinyl ether/maleic anhydride has a molecular weight (M.W.) of 700,000.

1.1.1. The compositions may further comprise an antibacterial agent. The antibacterial agent may be selected from halogenated diphenyl ether (e.g. triclosan), herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, zinc citrate, stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing.
1.1.2. Composition 1.1.1 wherein the antibacterial agent is triclosan.
1.1.3. Any of the preceding composition comprising triclosan and Zn²⁺ ion source, e.g., zinc citrate.
1.1.4. Any of the preceding compositions further comprising an anti-calculus agent.
1.1.5. Any of the preceding compositions further comprising an anti-calculus agent which is a polyphosphate, e.g., pyrophosphate, tripolyphosphate, or hexametaphosphate, e.g., in sodium salt form.
1.1.6. Any of the foregoing Compositions further comprising a fluoride ion source.
1.1.7. Composition 1.1.6 wherein the fluoride ion source is a soluble fluoride salt selected from stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'- tris(2-ethanol)-dihydrofluoride), ammonium fluoride, titanium fluoride, hexafluorosulfate, and combinations thereof.
1.1.8. Any of the foregoing Compositions further comprising a surfactant.
1.1.9. Any of the compositions further comprising an agent that interferes with or prevents bacterial attachment, e.g., solbrol or chitosan.
1.1.10. Any of the foregoing Compositions in the form of a toothpaste optionally further comprising one or more of one or more of water, abrasives, surfactants, foaming agents, vitamins, polymers, enzymes, humectants, thickeners, antimicrobial agents, preservatives, flavorings, colorings and/or combinations thereof.

The invention further comprises an oral care composition as described herein for use in a method of:
i. reducing or inhibit formation of dental caries,
ii. reducing, repairing or inhibiting early enamel lesions,
iii. reducing or inhibiting demineralization and promoting remineralization of the teeth,
iv. reducing hypersensitivity of the teeth,
v. reducing or inhibiting gingivitis,
vi. promoting healing of sores or cuts in the mouth,
vii. reducing levels of acid producing bacteria,
viii. increasing relative levels of arginolytic bacteria,
ix. inhibiting microbial biofilm formation in the oral cavity,
x. raising and/or maintaining plaque pH at levels of at least pH about 5.5 following sugar challenge,
xi. reducing plaque accumulation,
xii. treating, relieving or reducing dry mouth,
xiii. whitening teeth,
xiv. enhancing systemic health, including cardiovascular health, e.g., by reducing potential for systemic infection via the oral tissues,
xv. reducing erosion of the teeth,
xvi. immunizing the teeth against cariogenic bacteria, and/or
xvii. cleaning the teeth and oral cavity,
wherein the method comprises applying an effective amount of the composition to the oral cavity of a subject in need thereof.

In various embodiments of the Compositions of the invention, arginine is present in an amount of 0.5 wt. % to 20 wt. % of the total composition weight, about 1 wt. % to 10 wt. % of the total composition weight, for example 1.5 wt. %, 3.75 wt. %, 5 wt. %, or 7.5 wt. % of the total composition weight.

### Fluoride Ion Source

The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al.

Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof.

In certain embodiments, the oral care composition of the invention may also contain a source of fluoride ions or fluorine-providing ingredient in amounts sufficient to supply 25 ppm to 25,000 ppm of fluoride ions, generally at least 500 ppm, e.g., 500 to 2000 ppm, e.g., 1000 to 1600 ppm, e.g., 1450 ppm. The appropriate level of fluoride will depend on the particular application. A mouthwash, for example, would typically have 100 to 250 ppm fluoride. A toothpaste for general consumer use would typically have 1000 to 1500 ppm, with pediatric toothpaste having somewhat less. A dentifrice or coating for professional application could have as much as 5,000 or even 25,000 ppm fluoride.

Fluoride ion sources may be added to the compositions of the invention at a level of 0.01 wt. % to 10 wt. % in one embodiment or about 0.03 wt. % to 5 wt. %, and in another embodiment 0.1 wt. % to 1 wt. % by weight of the composition in another embodiment. Weights of fluoride salts to provide the appropriate level of fluoride ion will obviously vary based on the weight of the counter ion in the salt.

### Abrasives

The Compositions of the Invention may comprise a calcium phosphate abrasive, e.g., tricalcium phosphate (Ca₃(PO₄)₂), hydroxyapatite (Ca₁₀(PO₄)₆(OH)₂), or dicalcium phosphate dihydrate (CaHPO₄ • 2H₂O, also sometimes referred to herein as DiCal) or calcium pyrophosphate.

The compositions may include one or more additional abrasives, for example silica abrasives such as precipitated silicas having a mean particle size of up to about 20 microns, such as Zeodent 115®, marketed by J. M. Huber. Other useful abrasives also include sodium metaphosphate, potassium metaphosphate, aluminum silicate, calcined alumina, bentonite or other siliceous materials, or combinations thereof.

The silica abrasive polishing materials useful herein, as well as the other abrasives, generally have an average particle size of about 0.1 to about 30 microns, about 5 to about 15 microns. The silica abrasives can be from precipitated silica or silica gels, such as the silica xerogels described in U.S. Pat. No. 3,538,230, to Pader et al. and U.S. Pat. No. 3,862,307, to Digiulio. Particular silica xerogels are marketed under the trade name Syloid® by the W. R. Grace & Co., Davison Chemical Division. The precipitated silica materials include those marketed by the J. M. Huber Corp. under the trade name Zeodent®, including the silica carrying the designation Zeodent 115 and 119. These silica abrasives are described in U.S. Pat. No. 4,340,583, to Wason.

In certain embodiments, abrasive materials useful in the practice of the oral care compositions in accordance with the invention include silica gels and precipitated amorphous silica having an oil absorption value of less than 100 cc/100 g silica or 45 cc/100 g to 70 cc/100 g silica. Oil absorption values are measured using the ASTM Rub-Out Method D281. In certain embodiments, the silicas are colloidal particles having an average particle size of 3 microns to 12 microns, and 5 to 10 microns.

In particular embodiments, the abrasive materials comprise a large fraction of very small particles, e.g., having a d50 less than 5 microns, for example, small particle silica (SPS) having a d50 of 3-4 microns, for example Sorbosil AC43® (Ineos). Such small particles are particularly useful in formulations targeted at reducing hypersensitivity. The small particle component may be present in combination with a second larger particle abrasive. In certain embodiments, for example, the formulation comprises 3 to 8% SPS and 25 to 45% of a conventional abrasive.

Low oil absorption silica abrasives particularly useful in the practice of the invention are marketed under the trade designation Sylodent XWA® by Davison Chemical Division of W.R. Grace & Co., Baltimore, Md. 21203. Sylodent 650 XWA®, a silica hydrogel composed of particles of colloidal silica having a water content of 29% by weight averaging 7 to 10 microns in diameter, and an oil absorption of less than 70 cc/100 g of silica is an example of a low oil absorption silica abrasive useful in the practice of the present invention. The abrasive is present in the oral care composition of the present invention at a concentration of 10 to 60% by weight, in other embodiment 20 to 45% by weight, and in another embodiment 30 to 50% by weight.

### Agents to Increase the Amount of Foaming

The oral care compositions of the invention also may include an agent to increase the amount of foam that is produced when the oral cavity is brushed.

Illustrative examples of agents that increase the amount of foam include, but are not limited to polyoxyethylene and certain polymers including, but not limited to, alginate polymers.

The polyoxyethylene may increase the amount of foam and the thickness of the foam generated by the oral care carrier component of the present invention. Polyoxyethylene is also commonly known as polyethylene glycol ("PEG") or polyethylene oxide. The polyoxyethylenes suitable for this invention will have a molecular weight of 200,000 to 7,000,000. In one embodiment the molecular weight will be 600,000 to about 2,000,000 and in another embodiment 800,000 to 1,000,000. Polyox® is the trade name for the high molecular weight polyoxyethylene produced by Union Carbide.

The polyoxyethylene may be present in an amount of 1% to 90%, in one embodiment 5% to 50% and in another embodiment 10% to 20% by weight of the oral care carrier component of the oral care compositions of the present invention. The dosage of foaming agent in the oral care composition (i.e., a single dose) is 0.01 to 0.9 % by weight, 0.05 to 0.5% by weight, and in another embodiment 0.1 to 0.2 % by weight.

### Surfactants

Another agent optionally included in the oral care composition of the invention is a surfactant or a mixture of compatible surfactants. Suitable surfactants are those which are reasonably stable throughout a wide pH range, for example, anionic, cationic, nonionic or zwitterionic surfactants.

Suitable surfactants are described more fully, for example, in U.S. Pat. No. 3,959,458, to Agricola et al.; U.S. Pat. No. 3,937,807, to Haefele; and U.S. Pat. No. 4,051,234, to Gieske et al.

In certain embodiments, the anionic surfactants useful herein include the water-soluble salts of alkyl sulfates having 10 to 18 carbon atoms in the alkyl radical and the water-soluble salts of sulfonated monoglycerides of fatty acids having 10 to 18 carbon atoms. Sodium lauryl sulfate, sodium lauroyl sarcosinate and sodium coconut monoglyceride sulfonates are examples of anionic surfactants of this type. Mixtures of anionic surfactants may also be utilized.

In another embodiment, cationic surfactants useful in the present invention can be broadly defined as derivatives of aliphatic quaternary ammonium compounds having one long alkyl chain containing 8 to 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride, and mixtures thereof.

Illustrative cationic surfactants are the quaternary ammonium fluorides described in U.S. Pat. No. 3,535,421, to Briner et al. Certain cationic surfactants can also act as germicides in the compositions.

Illustrative nonionic surfactants that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

In certain embodiments, zwitterionic synthetic surfactants useful in the present invention can be broadly described as derivatives of aliphatic quaternary ammonium, phosphomium, and sulfonium compounds, in which the aliphatic radicals can be straight chain or branched, and wherein one of the aliphatic substituents contains about 8 to about 18 carbon atoms and one contains an anionic water-solubilizing group, e.g., carboxy, sulfonate, sulfate, phosphate or phosphonate. Illustrative examples of the surfactants suited for inclusion into the composition include, but are not limited to, sodium alkyl sulfate, sodium lauroyl sarcosinate, cocoamidopropyl betaine and polysorbate 20, and combinations thereof.

In a particular embodiment, the Composition of the Invention comprises an anionic surfactant, e.g., sodium lauryl sulfate.

The surfactant or mixtures of compatible surfactants can be present in the compositions of the present invention in 0.1% to 5.0%, in another embodiment 0.3% to 3.0% and in another embodiment 0.5% to 2.0% by weight of the total composition.

### Flavoring Agents

The oral care compositions of the invention may also include a flavoring agent. Flavoring agents which are used in the practice of the present invention include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent is incorporated in the oral composition at a concentration of 0.1 to 5% by weight and 0.5 to 1.5% by weight. The dosage of flavoring agent in the individual oral care composition dosage (i.e., a single dose) is 0.001 to 0.05% by weight and in another embodiment 0.005 to 0.015 % by weight.

### Chelating agents

The oral care compositions of the invention also may optionally include one or more chelating agents able to complex calcium found in the cell walls of the bacteria. Binding of this calcium weakens the bacterial cell wall and augments bacterial lysis.

Another group of agents suitable for use as chelating agents in the present invention are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide at least 1.0 wt. % pyrophosphate ions, 1.5 wt. % to 6 wt. %, 3.5 wt. % to 6 wt. % of such ions.

### Polymers

The oral care compositions of the invention also optionally include one or more polymers, such as polyethylene glycols, polyvinylmethyl ether maleic acid copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts.

Particularly when noncationic antibacterial agents or antibacterial agents, e.g., triclosan, are included in any of the dentifrice components, there is also preferably included from 0.05 to 5% of an agent which enhances the delivery and retention of the agents to, and retention thereof on oral surfaces. Such agents useful in the present invention are disclosed in U.S. Pat. Nos. 5,188,821 and 5,192,531; and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of 30,000 to 800,000. These copolymers are available for example as Gantrez. e.g., AN 139 (M.W. 500,000), AN 119 (M.W. 250,000) and preferably S-97 Pharmaceutical Grade (M.W. 700,000) available from ISP Technologies, Inc., Bound Brook, N.J. 08805. The enhancing agents when present are present in amounts of 0.05 to 3% by weight.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of 1,000 to 2,000,000, described in U.S. Pat. No. 4,842,847, Jun. 27, 1989 to Zahid, Another useful class of polymeric agents includes polyamino acids, particularly those containing proportions of anionic surface-active amino acids such as aspartic acid, glutamic acid and phosphoserine, as disclosed in U.S. Pat. No. 4,866,161 Sikes et al..

In preparing oral care compositions, it is sometimes necessary to add some thickening material to provide a desirable consistency or to stabilize or enhance the performance of the formulation. In certain embodiments, the thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate or finely divided silica can be used as component of the thickening composition to further improve the composition's texture. In certain embodiments, thickening agents in an amount of about 0.5% to about 5.0% by weight of the total composition are used.

### Enzymes

The oral care compositions of the invention may also optionally include one or more enzymes. Useful enzymes include any of the available proteases, glucanohydrolases, endoglycosidases, amylases, mutanases, lipases and mucinases or compatible mixtures thereof. In certain embodiments, the enzyme is a protease, dextranase, endoglycosidase and mutanase. In another embodiment, the enzyme is papain, endoglycosidase or a mixture of dextranase and mutanase. Additional enzymes suitable for use in the present invention are disclosed in U.S. Pat. No. 5,000,939 to Dring et al., U.S. Pat. No. 4,992,420; U.S. Pat. No. 4,355,022; U.S. Pat. No. 4,154,815; U.S. Pat. No. 4,058,595; U.S. Pat. No. 3,991,177; and U.S. Pat. No. 3,696,191. An enzyme of a mixture of several compatible enzymes in the current invention constitutes 0.002% to 2.0% in one embodiment or 0.05% to 1.5% in another embodiment or in yet another embodiment 0.1% to 0.5%.

### Water

Water may also be present in the oral compositions of the invention. Water, employed in the preparation of commercial oral compositions should be deionized and free of organic impurities. Water commonly makes up the balance of the compositions and includes 10% to 90%, 20% to 60% or 10% to 30% by weight of the oral compositions. This amount of water includes the free water which is added plus that amount which is introduced with other materials such as with sorbitol or any components of the invention.

### Humectants

Within certain embodiments of the oral compositions, it is also desirable to incorporate a humectant to prevent the composition from hardening upon exposure to air. Certain humectants can also impart desirable sweetness or flavor to dentifrice compositions. The humectant, on a pure humectant basis, generally includes 15% to 70% in one embodiment or 30% to 65% in another embodiment by weight of the dentifrice composition.

Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the toothpaste compositions herein.

In addition to the above described components, the embodiments of this invention can contain a variety of optional dentifrice ingredients some of which are described below. Optional ingredients include, for example, but are not limited to, adhesives, sudsing agents, flavoring agents, sweetening agents, additional antiplaque agents, abrasives, and coloring agents. These and other optional components are further described in U.S. Pat. No. 5,004,597, to Majeti; U.S. Pat. No. 3,959,458 to Agricola et al. and U.S. Pat. No. 3,937,807, to Haefele.

### Methods of Manufacture

The compositions of the present invention can be made using methods which are common in the oral product area.

In one illustrative embodiment, the Salts are made by neutralizing arginine in a gel phase with the conjugate acid and mixing to form Premix 1, adjusting the pH to the desired level, and then combining with the other ingredients. Actives such as, for example, vitamins, CPC, fluoride, abrasives, and any other desired active ingredients are added to Premix 1 and mixed to form Premix 2. Where the final product is a toothpaste, a toothpaste base, for example, dicalcium phosphate or silica, is added to Premix 2 and mixed. The final slurry is formed into an oral care product.

### Composition Use

The present invention in its method aspect involves applying to the oral cavity a safe and effective amount of the compositions described herein.

The compositions and methods according to the invention are useful to a method to protect the teeth by facilitating repair and remineralization, in particular to reduce or inhibit formation of dental caries, reduce or inhibit demineralization and promote remineralization of the teeth, reduce hypersensitivity of the teeth, and reduce, repair or inhibit early enamel lesions, e.g., as detected by quantitative light-induced fluorescence (QLF) or electronic caries monitor (ECM).

Quantitative Light-induced Fluorescence is a visible light fluorescence that can detect early lesions and longitudinally monitor the progression or regression. Normal teeth fluoresce in visible light; demineralized teeth do not or do so only to a lesser degree. The area of demineralization can be quantified and its progress monitored. Blue laser light is used to make the teeth auto fluoresce. Areas that have lost mineral have lower fluorescence and appear darker in comparison to a sound tooth surface. Software is used to quantify the fluorescence from a white spot or the area/volume associated with the lesion. Generally, subjects with existing white spot lesions are recruited as panelists. The measurements are performed in vivo with real teeth. The lesion area/volume is measured at the beginning of the clinical. The reduction (improvement) in lesion area/volume is measured at the end of 6 months of product use. The data is often reported as a percent improvement versus baseline.

Electrical Caries Monitoring is a technique used to measure mineral content of the tooth based on electrical resistance. Electrical conductance measurement exploits the fact that the fluid-filled tubules exposed upon demineralization and erosion of the enamel conduct electricity. As a tooth loses mineral, it becomes less resistive to electrical current due to increased porosity. An increase in the conductance of the patient's teeth therefore may indicate demineralization. Generally, studies are conducted of root surfaces with an existing lesion. The measurements are performed in vivo with real teeth. Changes in electrical resistance before and after 6 month treatments are made. In addition, a classical caries score for root surfaces is made using a tactile probe. The hardness is classified on a three point scale: hard, leathery, or soft. In this type of study, typically the results are reported as electrical resistance (higher number is better) for the ECM measurements and an improvement in hardness of the lesion based on the tactile probe score.

The Compositions of the Invention are thus useful in a method to reduce early lesions of the enamel (as measured by QLF or ECM) relative to a composition lacking effective amounts of fluorine and/or arginine.

The Compositions of the invention are additionally useful in methods to reduce harmful bacteria in the oral cavity, for example methods to reduce or inhibit gingivitis, reduce levels of acid producing bacteria, to increase relative levels of arginolytic bacteria, inhibit microbial biofilm formation in the oral cavity, raise and/or maintain plaque pH at levels of at least pH 5.5 following sugar challenge, reduce plaque accumulation, and/or clean the teeth and oral cavity.

Finally, by increasing the pH in the mouth and discouraging pathogenic bacteria, the Compositions of the Invention are useful to promote healing of sores or cuts in the mouth.

The compositions and methods according to the invention can be incorporated into oral compositions for the care of the mouth and teeth such as toothpastes, transparent pastes, gels, mouth rinses, sprays and chewing gum.

Enhancing oral health also provides benefits in systemic health, as the oral tissues can be gateways for systemic infections. Good oral health is associated with systemic health, including cardiovascular health. The compositions and methods of the invention provide particular benefits because basic amino acids, especially arginine, are sources of nitrogen which supply NO synthesis pathways and thus enhance microcirculation in the oral tissues. Providing a less acidic oral environment is also helpful in reducing gastric distress and creates an environment less favorable to Heliobacter, which is associated with gastric ulcers. Arginine in particular is required for high expression of specific immune cell receptors, for example T-cell receptors, so that arginine can enhance an effective immune response. The compositions and methods of the invention are thus useful to enhance systemic health, including cardiovascular health.

## Claims

1. An oral care composition comprising a salt of arginine and an acidic polymer which is a copolymer of methyl vinyl ether and maleic anhydride and has a molecular weight of 30,000 to 800,000.

2. The oral care composition of claim 1, wherein the salt is in solid form.

3. An oral care composition according to claim 1 or 2, for use in a method of:
xviii. reducing or inhibiting formation of dental caries,
xix. reducing, repairing or inhibiting early enamel lesions,
xx. reducing or inhibiting demineralization and promoting remineralization of the teeth,
xxi. reducing hypersensitivity of the teeth,
xxii. reducing or inhibiting gingivitis,
xxiii. promoting healing of sores or cuts in the mouth,
xxiv. reducing levels of acid producing bacteria,
xxv. increasing relative levels of arginolytic bacteria,
xxvi. inhibiting microbial biofilm formation in the oral cavity,
xxvii. raising and/or maintain plaque pH at levels of at least pH about 5.5 following sugar challenge,
xxviii. reducing plaque accumulation,
xxix. treating, relieving or reducing dry mouth,
xxx. whitening teeth,
xxxi. enhancing systemic health, including cardiovascular health,
xxxii. reducing erosion of the teeth,
xxxiii. immunizing the teeth against cariogenic bacteria, and/or
xxxiv. cleaning the teeth and oral cavity,
wherein the method comprises applying an effective amount of the composition to the oral cavity of a subject in need thereof.

## Patentansprüche

1. Mundpflegezusammensetzung umfassend ein Salz von Arginin und ein saures Polymer, das ein Copolymer von Methylvinylether und Maleinsäureanhydrid ist und ein Molekulargewicht von 30.000 bis 800.000 hat.

2. Mundpflegezusammensetzung nach Anspruch 1, worin das Salz in fester Form ist.

3. Mundpflegezusammensetzung nach Anspruch 1 oder 2 zur Verwendung in einem Verfahren von:
xviii. Bildung von Karies zu verringern oder zu verhindern,
xix. frühe Läsionen des Zahnschmelzes zu verringern, zu reparieren oder zu verhindern,
xx. Demineralisation zu verringern oder zu verhindern und eine Remineralisation der Zähne zu fördern,
xxi. Überempfindlichkeit der Zähne zu verringern,
xxii. Zahnfleischentzündung zu verringern oder zu verhindern,
xxiii. eine Heilung von wunden Stellen oder Schnittstellen im Mund zu fördern,
xxiv. Spiegel von Säure produzierenden Bakterien zu senken,
xxv. relative Spiegel von arginolytischen Bakterien zu erhöhen,
xxvi. Bildung eines mikrobiellen Biofilms in der Mundhöhle zu verhindern,
xxvii. den pH-Wert von Plaque auf einen Mindest-pH-Wer von etwa 5,5 nach einer Zuckerbelastung zu erhöhen und/oder auf diesem zu halten,
xxviii. Plaqueansammlung zu verringern,
xxix. Mundtrockenheit zu behandeln, zu lindern oder zu verringern,
xxx. Zähne zu weißen,
xxxi. Allgemeingesundheit, einschließlich der kardiovaskulären Gesundheit, zu verbessern,
xxxii. Erosion der Zähne zu verringern,
xxxiii. Zähne gegen kariogene Bakterien zu immunisieren und/oder
xxxiv. Zähne und Mundhöhle zu reinigen, worin das Verfahren das Anwenden einer wirksamen Menge der Zusammensetzung auf die Mundhöhle einer Person, die dieser Behandlung bedarf, umfasst.

## Revendications

1. Composition pour soins buccaux comprenant un sel d'arginine et un polymère acide qui est un copolymère d'éther méthylvinylique et d'anhydride maléique et a un poids moléculaire de 30 000 à 800 000.

2. Composition pour soins buccaux selon la revendication 1, dans laquelle le sel est sous forme solide.

3. Composition pour soins buccaux selon la revendication 1 ou 2, destinée à être utilisée dans un procédé de :
xviii. réduction ou inhibition de la formation de caries dentaires,
xix. réduction, réparation ou inhibition des lésions précoces de l'émail,
xx. réduction ou inhibition de la déminéralisation et promotion de la re-minéralisation des dents,
xxi. réduction de l'hypersensibilité des dents,
xxii. réduction ou inhibition de la gingivite,
xxiii. promotion de la cicatrisation des lésions ou des coupures de la bouche,
xxiv. réduction des niveaux de bactéries productrices d'acide,
xxv. augmentation des niveaux relatifs de bactéries arginolytiques,
xxvi. inhibition de la formation de biofilm microbien dans la cavité buccale,
xxvii. élévation et/ou maintien du pH de la plaque dentaire à des niveaux d'au moins environ 5,5 à la suite d'une attaque par du sucre,
xxviii. réduction de l'accumulation de plaque dentaire,
xxix. traitement, soulagement ou réduction de la bouche sèche,
xxx. blanchiment des dents,
xxxi. amélioration de la santé systémique, y compris la santé cardio-vasculaire,
xxxii. réduction de l'érosion des dents,
xxxiii. immunisation des dents contre les bactéries cariogènes, et/ou
xxxiv. nettoyage des dents et de la cavité buccale,
dans lequel le procédé comprend l'application d'une quantité efficace de la composition à la cavité buccale d'un sujet en ayant besoin.
